# EUROPEAN PATENT APPLICATION

(11) **EP 4 407 320 A1**
(43) Date of publication of application: **31.07.2024**
(21) Application number: 22871499.4
(22) Date of filing: 20.06.2022
(51) Int. Cl.: G01N 35/00, F25D 11/00

(54) **MICROFLUIDIC DETECTION SYSTEM FOR REFRIGERATOR, AND REFRIGERATOR**

(30) Priority: 26.09.2021 CN 202111131085
(71) Applicant: Qingdao Haier Refrigerator Co., Ltd., Laoshan District Qingdao Shandong 266101 (CN); Haier Smart Home Co., Ltd., Qingdao, Shandong 266101 (CN)
(72) Inventor: FEI, Bin, Qingdao, Shandong 266101 (CN); ZHAO, Bintang, Qingdao, Shandong 266101 (CN); SUN, Yongsheng, Qingdao, Shandong 266101 (CN)
(74) Representative: Winter, Brandl - Partnerschaft mbB
(86) International application number: PCT/CN2022/099817
(87) International publication number: WO 2023/045438

(57) **Abstract**

A microfluidic control detection system for a refrigerator and a refrigerator. The microfluidic control detection system comprises: a microfluidic biochip, comprising an inlet, a suction port, and a detection pool formed inside, where the inlet, the detection pool, and the suction port are sequentially interconnected through microchannels; a weighing platform, fixedly set on a support frame, and configured to measure weight of a sample contained in a sample cup placed on it; a buffer solution driving device, configured to deliver a predetermined amount of buffer solution matching the weight of the sample to the sample cup, thereby producing a sample solution by mixing the buffer solution with the sample in the sample cup; a bracket, configured to move in a controlled manner or an operable manner, driving the sample cup to move to a highest position where the sample liquid in the sample cup can contact the inlet of the microfluidic biochip; and a detection mechanism, configured to detect the detection pool to obtain predetermined detection parameters of the sample liquid that enters the detection pool through the inlet, offering high detection accuracy.

## Description

### CROSS-REFERENCE TO RELATED APPLICATION

The application claims priority from Chinese Patent Application No. 202111131085.3, filed September 26, 2021, entitled " Microfluidic Control Detection System and Refrigerator", all of which are incorporated herein by reference in their entirety.

### TECHNICAL FIELD

The present application relates to the field of refrigeration technology, and more particularly to a microfluidic control detection system and refrigerator.

### BACKGROUND

With the improvement of living standards, there is often a need in daily life to detect residues, viruses, nutritional elements, or other aspects of food ingredients either qualitatively or quantitatively. For instance, due to the misuse of pesticides, the fruits, vegetables, and agricultural products we buy daily may contain excessive levels of residues. If the excessive levels of residues in these foods are not discovered in time, ingestion can cause significant harm to the human body. Moreover, the currently advocated breastfeeding is best for infants only when the breast milk has normal nutritional value. However, illnesses, medication, surgery, or other situations of the nursing mother may lead to reduced nutritional element content in the secreted milk or even the presence of viruses, thereby affecting the infant's growth and health. However, the functionality of existing household appliances is relatively singular, and when there is a need to detect residues, viruses, nutritional elements, or other aspects of food ingredients, it necessitates the separate purchase of a dedicated detection device. This leads to a multitude of household appliances and types, occupying significant space, and does not align with the development trend of smart homes.

Existing microfluidic control detection systems can automatically add buffer solution and automatically oscillate to extract sample liquid. Before this, it's necessary to obtain weight of detection sample with high precision and then calculate required amount of buffer solution to add. After adding corresponding amount of buffer solution, a sample cup is oscillated for a certain time with an oscillator to extract residue components from the sample. However, in existing microfluidic control detection systems, a weighing scale is placed within a sample platform that holds the sample cup. To achieve automatic liquid suction of a chip and because a microfluidic biochip needs to be linked with a series of suction mechanisms and detection mechanisms, it's inconvenient for lifting, thus requiring the sample platform that holds the sample cup to move up and down. However, when the weighing scale moves with the sample platform, its weighing precision is greatly affected, which in turn affects the concentration of the sample liquid and consequently results in lower detection accuracy.

### SUMMARY

An object of a first aspect of the present application is to overcome at least one deficiency of the existing technology by providing a microfluidic control detection system for refrigerators that offers high weighing accuracy and accurate detection results.

A further object of a first aspect of the present application is to simplify the structure and control logic of the microfluidic control detection system.

An object of a second aspect of the present application is to provide a refrigerator equipped with the aforementioned microfluidic control detection system.

In accordance with a first aspect of the present application, the present application provides a microfluidic control detection system for a refrigerator, comprising:
a microfluidic biochip, comprising an inlet, a suction port, and a detection pool formed inside, where the inlet, the detection pool, and the suction port are sequentially interconnected through microchannels;
a weighing platform, fixedly set on a support frame, and configured to measure weight of a sample contained in a sample cup placed on it;
a buffer solution driving device, configured to deliver a predetermined amount of buffer solution matching the weight of the sample to the sample cup, thereby producing a sample solution by mixing the buffer solution with the sample in the sample cup;
a bracket, configured to move in a controlled manner or an operable manner, driving the sample cup to move to a highest position where the sample liquid in the sample cup can contact the inlet of the microfluidic biochip; and
a detection mechanism, configured to detect the detection pool to obtain predetermined detection parameters of the sample liquid that enters the detection pool through the inlet.

In an embodiment of the present application, the bracket is set above the weighing platform and comprises an annular frame that is fitted outside the sample cup; the bracket is configured to move in a controlled manner or an operable manner in an up and down direction, to use the annular frame to lift the sample cup off the weighing platform during upward movement, and to support the sample cup on the weighing platform and use the abutting effect between the sample cup and the weighing platform to detach the sample cup from the annular frame during downward movement to a lowest position.

In an embodiment of the present application, the annular frame is a circular ring-shaped frame, and the sample cup is a conical hollow cylinder with an outer diameter gradually increasing from bottom to top; and
an inner diameter of the annular frame is smaller than an outer diameter of a top end of the sample cup and larger than an outer diameter of a bottom end of the sample cup.

In an embodiment of the present application, the microfluidic control detection system further comprising second end of the connection channel is provided with a gradually expanding part, cross-sectional area of the gradually expanding part gradually increases from inside outwards; and
an oscillator, set on the bracket, configured to oscillate the sample cup after the bracket lifts the sample cup to a predetermined position where the sample cup is separated from the weighing platform, so that the buffer solution and the sample within the sample cup are thoroughly mixed; wherein
the predetermined position is lower than the highest position.

In an embodiment of the present application, the microfluidic control detection system further comprising:
a first power terminal, set on the support frame and electrically connected to a power supply of the microfluidic control detection system; and
a second power terminal, set on the bracket and electrically connected to the oscillator; and
the first power terminal and the second power terminal are configured to maintain elastic contact when the bracket is at a position between its lowest position where the bracket enables the sample cup supported on the weighing platform and the position where the bracket lifts the sample cup to the predetermined position, connecting the oscillator to the power supply, and configured to separate when the bracket lifts the sample cup from the predetermined position further upwards, disconnecting the oscillator from the power supply.

In an embodiment of the present application, the microfluidic control detection system further comprises a lifting mechanism for driving the bracket to move up and move down, which comprising:
a lifting motor, configured to output driving force; and
a drive screw, set vertically and connected to an output shaft of the lifting motor, to rotate under the drive of the lifting motor; wherein
the bracket is mounted on the drive screw and moves up and moves down along the drive screw as it rotates.

In an embodiment of the present application, the lifting mechanism further comprises:
a nut, mounted on the drive screw and connected to threads of the drive screw to move up and move down along the drive screw as it rotates; wherein
the bracket is fixedly connected to the nut, to drive the bracket to move up and move down through the nut.

In an embodiment of the present application, the lifting mechanism further comprises:
a guide rod, set beside the drive screw and extending vertically; wherein
the bracket is mounted on the guide rod, to guide the up movement and down movement of the bracket.

In an embodiment of the present application, the lifting mechanism further comprises:
a limit switch, set near the top of the lead screw, to cause the lifting motor to stop operating when the support frame moves up to touch the limit switch; and
a position of the limit switch is set so that when the lifting motor stops operating triggered by the limit switch, the sample cup on the bracket is at the highest position.

In an embodiment of the present application, the suction port located on a side surface parallel to a width direction and a length direction of the microfluidic biochip; and the microfluidic control detection system further comprises:
a sample liquid driving device, configured to be in communication with the suction port of the microfluidic biochip via a sealing connector after the microfluidic biochip is in its installed position, to prompt sample liquid in contact with the inlet to enter the microchannels and flow towards the detection pool under the control of the microfluidic biochip; and
a pressing mechanism, configured to apply pressure perpendicular to the side surface to the sealing connector after the microfluidic biochip is installed in its position, to form a fluidic seal connection between the sealing connector and the suction port.

In accordance with a second aspect of the present application, the present application provides a refrigerator, comprising the microfluidic control detection system according to any of the aforementioned technical solutions.

The microfluidic control detection system of the present application comprises a microfluidic biochip, a buffer solution driving device and a detection mechanism. Specially, the microfluidic control detection system further comprises a weighing platform fixed on a support frame and a bracket capable of moving the sample cup. During detection, users only need to place the sample cup on the weighing platform, which measures the weight of the sample. The buffer solution driving device adds an appropriate amount of buffer solution to the sample cup, and the bracket automatically moves the sample cup for sample addition to the microfluidic biochip, making the sampling operation convenient, time-saving, and labor-saving, leading to a good user experience. More importantly, since the weighing platform of the present application is fixed and does not move with movement of the bracket, the movement of the bracket does not affect weighing accuracy of the weighing platform, ensuring high-precision measurement of the sample's weight, thereby improving accuracy of detection results from the microfluidic biochip.

Further, inventors recognized that when the sample cup is weighed on the weighing platform, the bracket should be completely detached from and not touch the sample cup to avoid affecting weighing of the sample. After the weight of the sample has been measured, the bracket needs to hold the sample cup to move it together. That is, the bracket needs to have two states: releasing the sample cup and holding the sample cup, and it should be able to automatically switch between these two states according to a detection process. To achieve an object of switching between the two states, the common design approach before the present application was to equip the bracket with a clamping mechanism, which automatically switches between releasing the sample cup and holding the sample cup through control of the clamping mechanism's action. However, the applicants recognized that this traditional design approach is outdated and has many drawbacks. For example, the clamping mechanism increases structural complexity of the bracket and requires reserved space for action switch of the clamping mechanism to avoid interference or collision with other structures, which would increase volume of the microfluidic control detection system, making it unsuitable for refrigerators with limited space. Moreover, the holding of the sample cup, especially the release of the sample cup, needs to be highly synchronized with the detection process. That is, when the weighing platform needs to measure the weight of the sample, the clamping mechanism must be in a state of releasing the sample cup; only after the weighing platform has measured the weight of the sample can the clamping mechanism hold the sample cup. These requirements for timing precision of the clamping mechanism's state switching are very high. Any slight deviation or error accumulation could easily lead to disorder in the entire detection process and result in incorrect detection results.

To address this, the inventors attempted to break away from the traditional design approach and developed a completely new bracket structure. The bracket comprises an annular frame that is fitted outside the sample cup. As the bracket moves upwards, the annular frame naturally lifts the sample cup off the weighing platform; when the bracket moves down to a certain position, the sample cup is supported on the weighing platform, and as the bracket continues to move down to the lowest position, the abutting effect between the sample cup and the weighing platform causes the sample cup to detach from the annular frame, thus, the bracket does not affect weight measurement of the sample in any way. It is clear that the bracket of the present application naturally switches between lifting and releasing the sample cup during its lifting process, without the need for any lifting control programs or releasing control programs, making structure of the bracket simple, as well as control logic of the bracket.

The present application integrates the microfluidic control detection system into a refrigerator, fully leveraging the storage function of the refrigerator to make a detection process more convenient and facilitating linked control between the microfluidic detection system and the refrigerator. This higher level of automation meets the needs of smart homes.

Further details and the advantages and features of the present application will become clearer to those skilled in art from the detailed description of the specific embodiments in conjunction with the accompanying drawings below.

### BRIEF DESCRIPTION OF THE DRAWINGS

The subsequent text will describe some specific embodiments of the present application in a detailed but non-limiting manner with reference to drawings. The same reference numerals in the drawings denote the same or similar parts or components. It should be understood by those skilled in the art that these drawings are not necessarily drawn to scale. In the drawings:
FIG. 1 shows a schematic structural diagram of a microfluidic control detection system for a refrigerator according to an embodiment of the present application.
FIG.2 shows a schematic structural diagram of internal structure of a microfluidic control detection system according to an embodiment of the present application.
FIG.3 shows a schematic structural diagram of a microfluidic biochip according to an embodiment of the present application.
FIG.4 shows a schematic sectional view of a microfluidic biochip according to an embodiment of the present application.
FIG.5 shows a schematic structural diagram of a bracket, a weighing platform, and related structures according to an embodiment of the present application.
FIG.6 shows a schematic enlarged view of Part A in FIG.2.
FIG.7 shows a schematic sectional view of part of a microfluidic control detection system according to an embodiment of the present application.
FIG.8 shows a schematic enlarged view of Part B in FIG.7.
FIG.9 shows a schematic structural diagram of a refrigerator according to an embodiment of the present application.

### DETAILED DESCRIPTION

The present application initially provides a microfluidic control detection system for a refrigerator. The microfluidic control detection system of the present application is configured for the qualitative or quantitative detection of predetermined detection parameters of sample liquids. These predetermined detection parameters could include parameters indicating whether the amount of pesticide residue exceeds standards and/or the specific numerical value of the pesticide residue, parameters indicating whether nutritional elements meet standards and/or the specific content of nutritional elements, parameters for indicating whether specific harmful substances (such as specific viruses) exceed standards and/or specific content of specific harmful substances, among others.

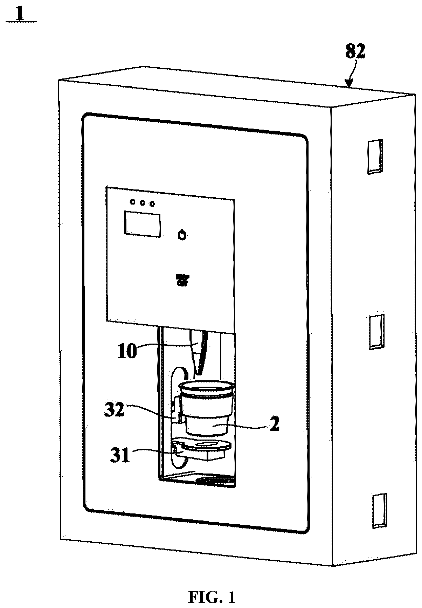

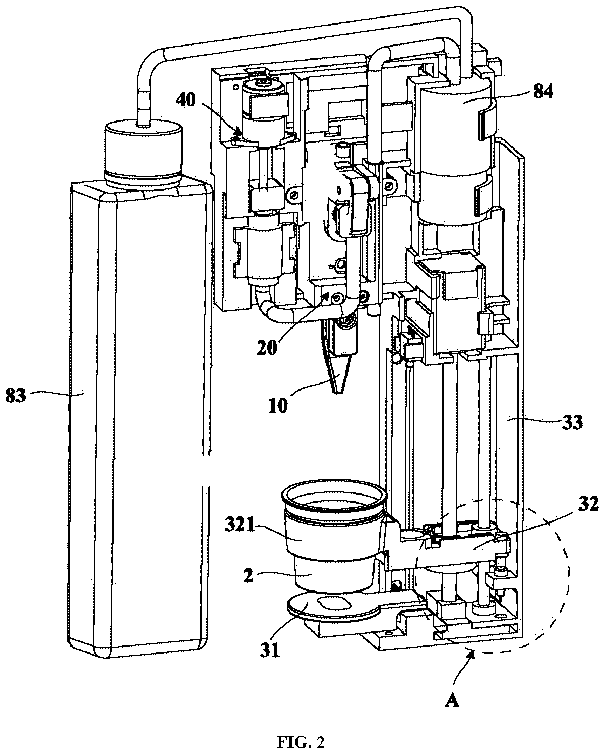

FIG. 1 shows a schematic structural diagram of a microfluidic control detection system for a refrigerator according to an embodiment of the present application, and FIG.2 shows a schematic structural diagram of internal structure of a microfluidic control detection system according to an embodiment of the present application. For ease of understanding, FIG.1 and FIG.2 also show a sample cup 2.

Referring to FIG. 1 to FIG.2, the microfluidic control detection system 1 related to the present application comprises a microfluidic biochip 10, a weighing platform 31, a buffer solution driving device 84, a bracket 32, and a detection mechanism 20.

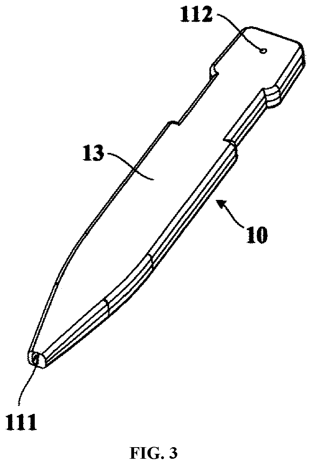

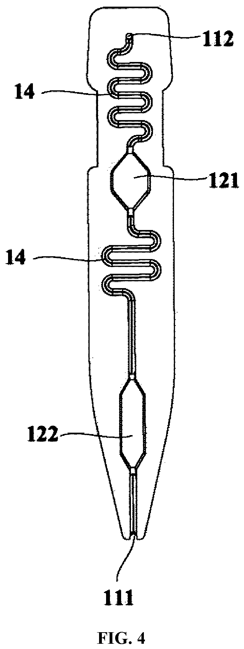

FIG.3 shows a schematic structural diagram of a microfluidic biochip according to an embodiment of the present application, and FIG.4 shows a schematic sectional view of a microfluidic biochip according to an embodiment of the present application. Referring to FIG.3 and FIG.4, the microfluidic biochip 10 comprises an inlet 111, a suction port 112, and a detection pool 121 formed inside the microfluidic biochip 10. The inlet 111, detection pool 121, and suction port 112 are sequentially interconnected through microchannels 14, thus forming a main channel. Specifically, the inlet 111 can be located on an end face of the microfluidic biochip 10 to facilitate contact with sample liquid. It can be understood that the microchannels referred to in the present application are fine or capillary channels with a cross-sectional flow area within a predetermined size range, to ensure the microchannels have appropriate capacity to retain liquid inside.

The weighing platform 31 is fixedly set on a support frame 33 and configured to measure weight of a sample contained in a sample cup 2 placed on it. It is understandable that the weighing platform 31 can measure combined weight of the sample cup 2 and the sample contained therein, subtracting weight of the sample cup 2 itself to obtain the weight of the sample. The weighing platform 31 can also be configured to directly detect the weight of the sample contained in the sample cup 2, such as through tare measurement.

The buffer solution driving device 84 is configured to deliver a predetermined amount of buffer solution matching the weight of the sample to the sample cup 2, thereby producing sample liquid by mixing the buffer solution with the sample in the sample cup 2.

The bracket 32 is configured to move in a controlled manner or operable manner, driving the sample cup 2 to move to a highest position where the sample liquid in the sample cup 2 can contact the inlet of the microfluidic biochip 10.

The detection mechanism 20 is configured to detect the detection pool 121 to obtain predetermined detection parameters of the sample liquid. Specifically, detection reagents can be pre-placed in the detection pool 121, or can be manually or automatically added to the detection pool 121, so that after a reaction of the sample liquid with the detection reagents inside the detection pool 121, the detection mechanism 20 performs detection on the detection pool 121.

The microfluidic control detection system 1 of the present application specially comprises a weighing platform 31 fixed on a support frame 33 and a bracket 32 capable of moving the sample cup 2. During detection, users only need to place the sample cup 2 on the weighing platform 31, which measures the weight of the sample. The buffer solution driving device 84 adds an appropriate amount of buffer solution to the sample cup 2, and the bracket 32 automatically moves the sample cup 2 for sample addition to the microfluidic biochip 10, making the sampling operation convenient, time-saving, and labor-saving, leading to a good user experience. More importantly, since the weighing platform 31 of the present application is fixed and does not move with movement of the bracket 32, the movement of the bracket 32 does not affect weighing accuracy of the weighing platform 31, ensuring high-precision measurement of the sample's weight, thereby improving accuracy of detection results from the microfluidic biochip 10.

It is understandable to those skilled in art that when the microfluidic control detection system is used for detecting different predetermined detection parameters, the specific choices of the microfluidic biochip 10 and the detection mechanism 20 might also vary. For example, when the microfluidic control detection system is used for pesticide residue detection, the microfluidic biochip 10 it contains could be a microfluidic pesticide detection chip capable of providing conditions for pesticide liquid detection, and the detection mechanism 20 it contains could be a pesticide detection mechanism capable of detecting pesticide parameters in the pesticide liquid.

In a specific embodiment, when the detection mechanism 20 is a pesticide detection mechanism for detecting pesticide parameters in pesticide liquid, a quick qualitative detection of whether pesticide residue in sample liquid exceeds standards can be conducted using the enzyme inhibition rate method. In this case, the microfluidic biochip 10 further comprises a reaction pool 122 formed inside it. The reaction pool 122 is located on the main channel formed by sequentially connecting the inlet 111, the detection pool 121, and the connecting port 112 and is connected between the inlet 111 and the detection pool 121, allowing sample liquid to react with reaction reagents in the reaction pool 122 before flowing into the detection pool 121. The reaction pool 122 is connected to both the inlet 111 and the detection pool 121 through microchannels 14. The reaction reagent and detection reagent used for pesticide detection can be enzyme reagents and chromogenic agents, respectively. The reaction pool 122 is used for sample liquid to react with the enzyme reagent inside it; the sample liquid that has reacted with the enzyme reagent flows into the detection pool 121 and reacts with the chromogenic agent in the detection pool 121. The detection mechanism 20 can be selected as a photoelectric detection mechanism, which may comprise structures such as a light source, a photosensitive element, a heating plate, and a thermostat.

Inventors recognized that when the sample cup 2 is weighed on the weighing platform 31, the bracket 32 should be completely detached from and not touch the sample cup 2 to avoid affecting weighing of the sample. After the weight of the sample has been measured, the bracket 32 needs to hold the sample cup 2 to move it together. That is, the bracket 32 needs to have two states: releasing the sample cup and holding the sample cup, and it should be able to automatically switch between these two states according to a detection process. To achieve an object of switching between the two states, the common design approach before the present application was to equip the bracket with a clamping mechanism, which automatically switches between releasing the sample cup and holding the sample cup through control of the clamping mechanism's action. However, the applicants recognized that this traditional design approach is outdated and has many drawbacks. For example, the clamping mechanism increases structural complexity of the bracket and requires reserved space for action switch of the clamping mechanism to avoid interference or collision with other structures, which would increase volume of the microfluidic control detection system, making it unsuitable for refrigerators with limited space. Moreover, the holding of the sample cup, especially the release of the sample cup, needs to be highly synchronized with the detection process. That is, when the weighing platform needs to measure the weight of the sample, the clamping mechanism must be in a state of releasing the sample cup; only after the weighing platform has measured the weight of the sample can the clamping mechanism hold the sample cup. These requirements for timing precision of the clamping mechanism's state switching are very high. Any slight deviation or error accumulation could easily lead to disorder in the entire detection process and result in incorrect detection results.

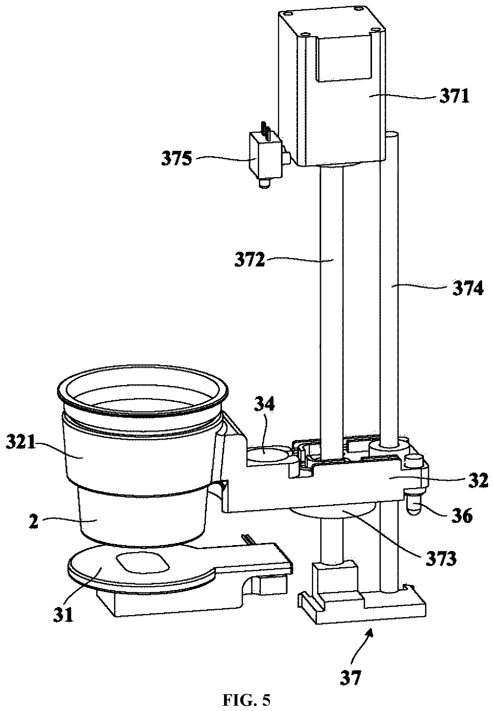

To address this, the inventors attempted to break away from the traditional design approach and developed a completely new bracket structure. FIG.5 shows a schematic structural diagram of the bracket, weighing platform, and their related structures according to an embodiment of the present application. In some embodiments, the bracket 32 is set above the weighing platform 31 and comprises an annular frame 321 that is fitted outside the sample cup 2. The bracket 32 is configured to move controllably or operably in an up and down direction, using the annular frame 321 to lift the sample cup 2 off the weighing platform 31 as it moves upwards, and during its downward movement to a lowest position, it allows the sample cup 2 to be supported on the weighing platform 31 and uses an abutting effect between the sample cup 2 and the weighing platform 31 to detach the sample cup 2 from the annular frame 321.

That is, as the bracket 32 moves upwards, the annular frame 321 naturally lifts the sample cup 2 off the weighing platform 31; when the bracket 32 moves down to a certain position, the sample cup 2 is supported on the weighing platform 31, and as the bracket 32 continues to move down to the lowest position, the abutting effect between the sample cup 2 and the weighing platform 31 causes the sample cup 2 to detach from the annular frame 321, thus, the bracket 32 does not affect weight measurement of the sample in any way. It is clear that the bracket 32 of the present application naturally switches between lifting and releasing the sample cup 2 during its lifting process, without the need for any lifting control programs or releasing control programs, making structure of the bracket 32 simple, as well as control logic of the bracket 32.

In some embodiments, the annular frame 321 is a circular ring-shaped frame, and the sample cup 2 is a conical hollow cylinder with an outer diameter gradually increasing from bottom to top. An inner diameter of the annular frame 321 is smaller than an outer diameter of a top end of the sample cup 2 and larger than an outer diameter of a bottom end of the sample cup 2. Thus, as the bracket 32 moves up from its lowest position, since the inner diameter of the annular frame 321 is larger than the outer diameter of the bottom end of the sample cup 2, the annular frame 321 does not affect the sample cup 2 at that moment. When the annular frame 321 moves up or down relative to and touch the upper part or top end of the sample cup 2 resting on the weighing platform 31, the sample cup 2 gets caught on the annular frame 321 and is lifted by the annular frame 321 as the annular frame 321 continues to move upwards. When the bracket 32 moves downwards, the sample cup 2 gradually descends, and when the sample cup 2 touches and is supported by the weighing platform 31, it stays stationary under support of the weighing platform 31 without moving any further, while the annular frame 321 continues to move down until the bracket 32 reaches its lowest position. During the moving process, since the inner diameter of the annular frame 321 is larger than the outer diameter of the bottom end of the sample cup 2, the annular frame 321 gradually separates from the sample cup 2 until it is completely detached from the sample cup 2.

In some alternative embodiments, the annular frame 321 can also be other shapes of closed frames, and the sample cup 2 can also be hollow cylindrical or other shapes. In this case, similar structures such as hooks can be designed on the annular frame 321 or the sample cup 2 so that the annular frame 321 naturally lifts the sample cup 2 or releases the sample cup 2 during the lifting process.

In some embodiments, the microfluidic control detection system 1 of the present application also comprises an oscillator 34, which is set on the bracket 32. The oscillator 34 is configured to oscillate the sample cup 2 after the bracket 32 lifts the sample cup 2 to a predetermined position where the sample cup 2 is separated from the weighing platform 31. This oscillation ensures that the buffer solution and the sample within the sample cup 2 are thoroughly mixed, thus allowing substances to be detected to dissolve fully into the buffer solution, resulting in a sample liquid of appropriate concentration. Furthermore, the aforementioned predetermined position is lower than the aforementioned highest position. That is, a position of the sample cup 2 during oscillation is lower than a position of the sample cup 2 where the sample liquid in the sample cup 2 contacts the inlet 111 of the microfluidic biochip 10.

In the present application, by placing the oscillator 34 on the bracket 32, and with the sample cup 2 being detached from the weighing platform 31 during oscillation of the sample cup 2 by the oscillator 34, the oscillation by the oscillator 34 does not affect the weighing platform 31. This design avoids a problem encountered in existing technologies where setting an oscillator together with a weighing platform could lead to reduced accuracy or even damage to the weighing platform due to prolonged oscillation.

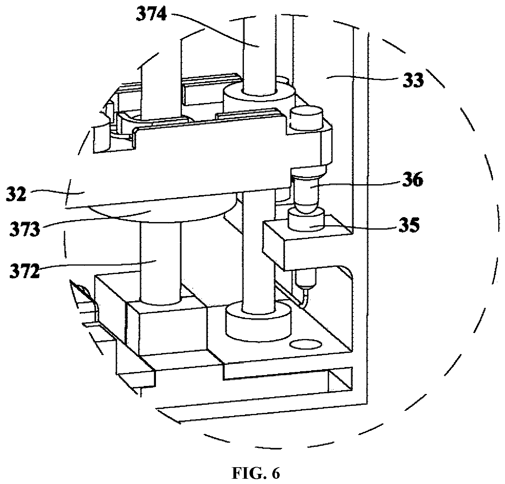

FIG.6 shows a schematic enlarged view of Part A in FIG.2. Referring to FIG.5 and FIG.6, in some embodiments, the microfluidic control detection system 1 of the present application also comprises a first power terminal 35 and a second power terminal 36. The first power terminal 35 is set on the support frame 33 and electrically connected to a power supply of the microfluidic control detection system 1. The second power terminal 36 is set on the bracket 32 and electrically connected to the oscillator 34.

Furthermore, the first power terminal 35 and the second power terminal 36 are configured to maintain elastic contact when the bracket 32 is at a position between its lowest position where the bracket 32 enables the sample cup 2 supported on the weighing platform 31 and the position where the bracket 32 lifts the sample cup 2 to the aforementioned predetermined position, thus connecting the oscillator 34 to the power supply. The first power terminal 35 and the second power terminal 35 are configured to separate, when the bracket 32 lifts the sample cup 2 from the predetermined position further upwards, thus disconnecting the oscillator 34 from the power supply. That is, throughout the complete lifting process of the bracket 32, the first power terminal 35 and the second power terminal 36 can elastically stretch and maintain contact only within a short travel range below the height of the predetermined position where the sample cup 2 is located, thus powering the oscillator 34. When the oscillator 34 has completed oscillating and the bracket 32 continues to move up, the first power terminal 35 and the second power terminal 36 separate, eliminating the need for cables sliding up and down. In this embodiment, the cable between the first power terminal 35 and the power source is stationary and of appropriate length, avoiding any messy situations. The cable between the second power terminal 36 and the oscillator 34 can be hidden within the bracket 32, not moving relative to the bracket 32 and also avoiding any messy situations. Specifically, the first power terminal 35 can be set at the bottom of the support frame 33, and the second power terminal 36 can protrude downwards from the bracket 32.

In other embodiments, the first power terminal 35 and the second power terminal 36 can also be configured to disconnect when the bracket 32 is at its lowest position supporting the sample cup 2 on the weighing platform 31 to disconnect the oscillator 34 from the power supply and maintain elastic contact when the bracket 32 lifts the sample cup 2 to the predetermined position to connect the oscillator 34 to the power supply. That is, throughout the complete lifting process of the bracket 32, only within a short travel range above the height of the predetermined position where the sample cup 2 is located, the first power terminal 35 and the second power terminal 36 can elastically stretch and maintain contact, thus powering the oscillator 34. Before the oscillator 34 starts oscillating, the bracket 32 is still relatively low, and the first power terminal 35 and the second power terminal 36 are separated, eliminating the need for cables sliding up and down. In this embodiment, the cable between the first power terminal 35 and the power source is also stationary and of appropriate length, avoiding any messy situations. The cable between the second power terminal 36 and the oscillator 34 can be hidden within the bracket 32, not moving relative to the bracket 32 and also avoiding any messy situations. Specifically, the first power terminal 35 can be set at the top of the support frame 33, and the second power terminal 36 can protrude upwards from the bracket 32.

In some embodiments, the microfluidic control detection system 1 of the present application also comprises a lifting mechanism 37 for driving the bracket 32 to move up and move down. The lifting mechanism 37 specifically may comprise a lifting motor 371 and a drive screw 372. The lifting motor 371 is configured to output driving force. The drive screw 372 (which in one embodiment can be configured as a lead screw) is set vertically and connected to an output shaft of the lifting motor 371 to rotate under the drive of the lifting motor 371. The bracket 32 is mounted on the drive screw 372 and moves up and moves down along the drive screw 372 as it rotates.

Furthermore, threads may be set on the drive screw 372, and the bracket 32 can directly connect to the threads of the drive screw 372 to move along the drive screw 372 as the drive screw rotates. The bracket 32 can also be indirectly connected to the drive screw 372 through other structures. For example, in a preferred embodiment, the lifting mechanism 60 comprises a nut 373, which is mounted on the drive screw 372 and connected to the threads of the drive screw 372 to move up and move down along the drive screw 372 as it rotates. The bracket 32 is fixedly connected to the nut 373 to drive the bracket 32 to move up and move down through the nut 373.

In some embodiments, the lifting mechanism 37 also comprises a guide rod 374, set beside the drive screw 372 and extending vertically. The bracket 32 is mounted on the guide rod 374 to guide the up movement and down movement of the bracket 32, avoiding offset, rotation, or jamming of the bracket 32 during movement and improving the smoothness of the movement of the bracket 32.

In some embodiments, the lifting mechanism 37 also comprises a limit switch 375, set near the top of the drive screw 372 to cause the lifting motor 371 to stop operating when the bracket 32 moves up to touch the limit switch 375. A position of the limit switch 375 is set so that when the lifting motor 371 stops operating triggered by the limit switch 375, the sample cup 2 on the bracket 32 is at the aforementioned highest position, i.e., a position where the sample liquid in the sample cup 2 contacts the inlet 111 of the microfluidic biochip 10. The present application uses the limit switch 375 to precisely locate a detection position of the bracket 32, avoiding the issue of the bracket 32 moving beyond its detection position and causing damage to the bracket 32, microfluidic biochip 10, and other structures.

In some embodiments, the suction port 112 is located on a side surface 13 that is parallel to the a width direction and a length direction of the microfluidic biochip 10. It can be understood that the microfluidic biochip 10 usually has a thin, flat shape, where the side surface 13 refers to a side surface parallel to both the width direction and the length direction of the microfluidic biochip 10, perpendicular to a thickness direction of the microfluidic biochip 10. The end face of the microfluidic biochip 10 refers to a surface located at an end of the microfluidic biochip 10.

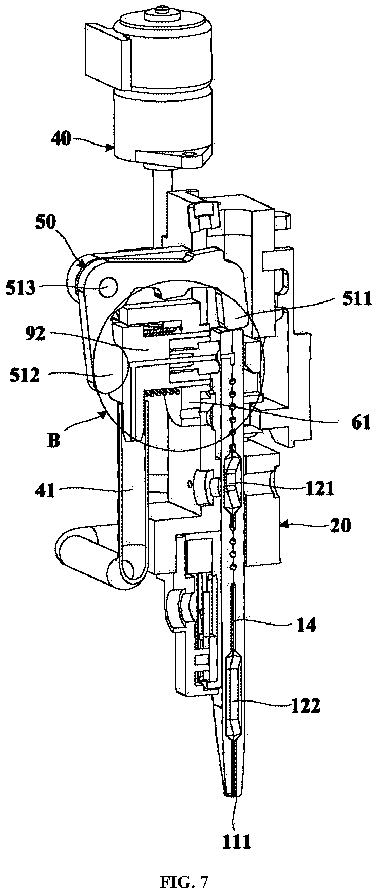

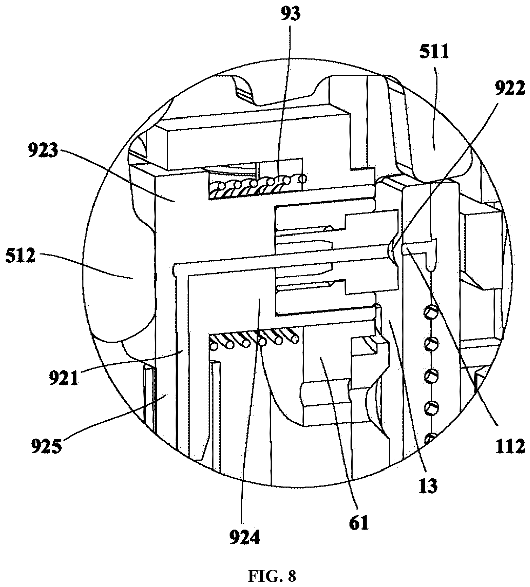

FIG.7 shows a schematic sectional view of a part of the microfluidic control detection system according to an embodiment of the present application, and FIG.8 shows a schematic enlarged view of Part B in FIG.7. Furthermore, the microfluidic control detection system 1 of the present application also comprises a sample liquid driving device 40 and a pressing mechanism 50. The sample liquid driving device 40 is configured to communicate with the suction port 112 of the microfluidic biochip 10 via a sealing connector 92 once the microfluidic biochip 10 is in its installed position, thereby, under the control of the microfluidic biochip 10, prompting sample liquid in contact with the inlet 111 to enter the microchannels 14 and flow towards the detection pool 121 through the microchannels 14. The pressing mechanism is configured to apply pressure perpendicular to the side surface 13 to the sealing connector 92 after the installation of the microfluidic biochip 10 in its position, to form a fluidic seal connection between the sealing connector 92 and the suction port 112.

In the present application, the suction port 112 of the microfluidic biochip 10 is located on a side surface 13 parallel to a width direction of the microfluidic biochip 10 and a length direction of the microfluidic biochip 10, which offers a much larger area compared to an end face of the microfluidic biochip 10. This is advantageous for forming a relatively large sealing interface around the suction port 112, thereby significantly improving sealing performance between the sample liquid driving device 40 and the suction port 112, preventing liquid leakage, air leakage and enhancing the precision of sample introduction control by the sample liquid driving device 40.

Moreover, the pressing mechanism can apply pressure perpendicular to the side surface 13 of the microfluidic biochip 10 to a sealing connector 92 located between the sample liquid driving device 40 and the suction port 112 of the microfluidic biochip 10 after the microfluidic biochip 10 is installed in its position. The pressure ensures the sealing connector 92 tightly presses against the side surface 13 of the microfluidic biochip 10, further improving sealing performance of sealing interface formed between the sealing connector 92 and the side surface around the suction port 112. Furthermore, the pressing mechanism automatically applies pressure to the sealing connector 92 upon the installation of the microfluidic biochip 10, achieving coupling between chip installation and sealing connection. Users do not need to perform any actions other than installing the chip, simplifying detection operations and enhancing user experience.

In some embodiments, an internal part of the sealing connector 92 forms a through connection channel 921. A first end of the connection channel 921 communicates with the sample liquid driving device 40, and after the microfluidic biochip 10 is installed in its position, a second end of the connection channel 921 communicates with the suction port 112 of the microfluidic biochip 10. Thus, the sample liquid driving device 40 can be in fluid communication with the suction port 112 of the microfluidic biochip 10 through the connection channel 921.

Furthermore, the second end of the connection channel 921 is provided with a gradually expanding part 922, whose cross-sectional area gradually increases from inside outwards, enabling the second end of the connection channel 921 to have a larger opening area. After the microfluidic biochip 10 is installed in its position, the gradually expanding part 922 covers the suction port 112 of the microfluidic biochip 10, thereby forming fluid communication between the connection channel 921 and the suction port 112. It is understandable that there may be positional deviations when installing the microfluidic biochip 10, and configuration of the expanding part 922 allows for certain positional offsets between the connection channel 921 and the suction port 112, thus ensuring stable fluid communication between the suction port 112 and the connection channel 921 within an allowable range of installation errors. Simultaneously, after the microfluidic biochip 10 is installed in its position, a relatively large sealing interface is formed between the sealing connector 92 and the side surface 13, resulting in better sealing performance.

In some embodiments, the pressing mechanism comprises a lever that rotates around a fixed rotation axis 513. The lever comprises a first end 511 and a second end 512. The microfluidic biochip 10 is configured to abut against the first end 511 of the lever during installation, causing the lever to rotate around its rotation axis 513 until the microfluidic biochip 10 is installed in its position. As the lever rotates around its rotation axis 513, the second end 512 of the lever abuts against the sealing connector 92 and prompts the sealing connector 92 to move in a direction gradually approaching the side surface 13 of the microfluidic biochip 10, thereby pressing the sealing connector 92 against the side surface 13. Once the microfluidic biochip 10 is installed in its position, the lever no longer rotates, and the second end 512 of the lever maintains its abutting action against the sealing connector 92, thus keeping the sealing connector 92 pressed against the side surface 13.

Leveraging the principle of levers, the first end 511 and the second end 512 of the lever can be positioned at different locations and can extend in different directions. Therefore, movement direction of the first end 511 of the lever15 and movement direction of the second end 512 of the lever can be varied according to their extension directions. This enables the coupling of chip installation and sealing connection even if the installation direction of the microfluidic biochip 10 and the direction in which the sealing connector 92 presses against the side surface 13 of the microfluidic biochip 10 are perpendicular to each other. Additionally, structure of lever is very simple, not complicating structure of the microfluidic detection system 1.

In some embodiments, the microfluidic control detection system 1 also comprises a first bracket 61 configured to support the sealing connector 92. The sealing connector 92 comprises a cap part 923 and a rod part 924, which are perpendicular to each other. The rod part 924 can be slidably inserted into the first bracket 61 to form a fluid connection with the suction port 112. A first spring 93 is fitted around the exterior of the rod part 924, with one end of the first spring 93 abutting against the inner side of the cap part 923 towards the rod part 924, and another end of the first spring 94 abutting against the first bracket 61. The second end of the lever abuts against the outer side of the cap part 923, away from the rod part 924.

Furthermore, the first end 511 of the lever can be located near the installation position of the microfluidic biochip 10 to abut against an end face of the microfluidic biochip 10 during its installation process, and rotate as the microfluidic biochip 10 is installed. During the rotation of the lever 51, its second end 512 applies pressure towards the sealing connector 92 in the direction towards the microfluidic biochip 10, causing the first spring 93 to compress. When the microfluidic biochip 10 is being disassembled, abutting action of the microfluidic biochip 10 against the first end 511 of the lever decreases or disappears, under elastic deformation recovery force of the first spring 93, the sealing connector 92 applies a force on the second end 512 of the lever in the direction away from the microfluidic biochip 10, causing the lever to rotate in the opposite direction, and the sealing connector 92 to detach from the side surface 13 of the microfluidic biochip 10. Simultaneously, the first end 511 of the lever can also generate a force that facilitates the popping out of the microfluidic biochip 10, aiding in smooth disassembly of the microfluidic biochip 10. Specifically, the lever can be roughly U-shaped.

In some embodiments, the sample liquid driving device 40 is connected to a suction pipeline 41 that is in fluid communication with it. The cap part 923 comprises a protruding extension in the form of a connecting column 925, which is inserted into the suction pipeline 41 to ensure that the internal connection channel 921 of the connecting column 925 is in sealed communication with the suction pipeline 41. Since the connecting column 925 is connected to the suction pipeline 41 through an insertion method, a large contact area between them ensures good sealing performance.

In some embodiments, the sample liquid driving device 40 may be a micro injection pump that creates a vacuum within a main channel by drawing air outwards, thereby allowing sample liquid in contact with the inlet port 111 to enter the main channel under the influence of the vacuum. Specifically, the sample liquid driving device 40 comprises a drive motor, a vertically extending syringe, a lead screw, a slider, and a piston, among other components.

In some embodiments, the microfluidic detection system 1 may comprise a housing 82. The housing 82 has an operation platform open towards front side of the housing 82, facilitating user operations such as placing the sample cup 2 or removing the sample cup 2.

In some embodiments, the microfluidic detection system 1 may comprise a buffer solution bottle 83. The buffer solution bottle 83, located within the housing 82, is configured to contain a buffer solution. The buffer solution driving device 84, also located within the housing 82 and connected to the buffer solution bottle 83, is controlled to drive the buffer solution from the buffer solution bottle 83 into a sample cup 2. Mixture of buffer solution and sample in the sample cup 2 produces sample liquid. Specifically, the buffer solution driving device 84 can be a peristaltic pump, diaphragm pump, or another suitable type of driving device.

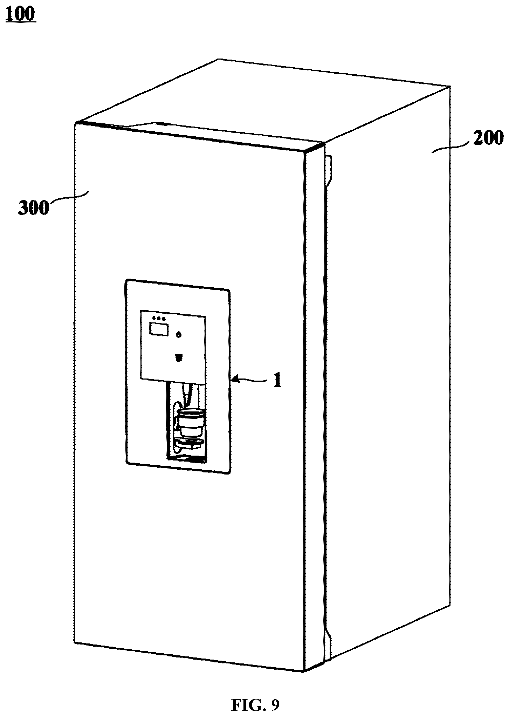

The present application also introduces a refrigerator, as illustrated in FIG.9, illustrating a schematic structural diagram of a refrigerator according to an embodiment of the present application. The refrigerator 100 incorporates the microfluidic detection system 1 involved in any of the aforementioned embodiments, integrating the microfluidic detection system 1 with the refrigerator 100. Given the high frequency of use of refrigerators 100 in daily life and their primary function for storing food ingredients, integrating the microfluidic detection system 1 into the refrigerator 100 facilitates users in conducting detection operations on food ingredient samples using the microfluidic detection system 1.

By integrating the microfluidic detection system 1 into the refrigerator 100, the present application fully leverages storage function of the refrigerator 100, making a detection process more convenient. It also facilitates the interlinked control between the microfluidic detection system 1 and the refrigerator 100, achieving a high level of intelligence that meets needs of smart homes.

Additionally, the refrigerator 100 comprises a cabinet 200 and a door 300. The cabinet 200 defines a storage space, and the door 300 is connected to the cabinet 200 to open and/or close the storage space. Preferably, the microfluidic detection system 1 is mounted on the door 300, which is not only convenient for operation but also does not occupy an original storage space inside the cabinet 200, thus not affecting storage capacity of the refrigerator 100 itself.

The refrigerator 100 mentioned in the present application is broadly defined to include not only the conventional narrow sense of refrigerators but also storage devices with refrigeration, freezing, or other storage functions, such as refrigeration boxes, freezers, etc.

It should be understood by those skilled in the art that the terms "up," "down," "front," "back," "top," "bottom," and other directional or positional terms used in the embodiments of the present application are based on an actual usage state of the microfluidic detection system 1 and the refrigerator 100. These terms are merely for the convenience of describing and understanding the technical solutions of the present application and should not be interpreted as limiting the present application to specific orientations or configurations.

Thus, it should be recognized by those skilled in art that although this document has thoroughly presented and described several exemplary embodiments of the present application, many other variations or modifications that are in accordance with the principles of the present application can be directly determined or derived from the content disclosed here without departing from the spirit and scope of the present application. Therefore, the scope of the present application should be understood and recognized to cover all such other variations or modifications.

## Claims

1. A microfluidic control detection system for a refrigerator, **characterized in that** the microfluidic control detection system comprises:
a microfluidic biochip, comprising an inlet, a suction port, and a detection pool formed inside, where the inlet, the detection pool, and the suction port are sequentially interconnected through microchannels;
a weighing platform, fixedly set on a support frame, and configured to measure weight of a sample contained in a sample cup placed on it;
a buffer solution driving device, configured to deliver a predetermined amount of buffer solution matching the weight of the sample to the sample cup, thereby producing a sample solution by mixing the buffer solution with the sample in the sample cup;
a bracket, configured to move in a controlled manner or an operable manner, driving the sample cup to move to a highest position where the sample liquid in the sample cup can contact the inlet of the microfluidic biochip; and
a detection mechanism, configured to detect the detection pool to obtain predetermined detection parameters of the sample liquid that enters the detection pool through the inlet.

2. The microfluidic control detection system according to claim 1, **characterized in that**:
the bracket is set above the weighing platform and comprises an annular frame that is fitted outside the sample cup; the bracket is configured to move in a controlled manner or an operable manner in an up and down direction, to use the annular frame to lift the sample cup off the weighing platform during upward movement, and to support the sample cup on the weighing platform and use the abutting effect between the sample cup and the weighing platform to detach the sample cup from the annular frame during downward movement to a lowest position.

3. The microfluidic control detection system according to claim 2, **characterized in that**:
the annular frame is a circular ring-shaped frame, and the sample cup is a conical hollow cylinder with an outer diameter gradually increasing from bottom to top; and
an inner diameter of the annular frame is smaller than an outer diameter of a top end of the sample cup and larger than an outer diameter of a bottom end of the sample cup.

4. The microfluidic control detection system according to claim 2 or 3, **characterized in that** the microfluidic control detection system further comprises:
an oscillator, set on the bracket, configured to oscillate the sample cup after the bracket lifts the sample cup to a predetermined position where the sample cup is separated from the weighing platform, so that the buffer solution and the sample within the sample cup are thoroughly mixed; wherein
the predetermined position is lower than the highest position.

5. The microfluidic control detection system according to claim 4, **characterized in that** the microfluidic control detection system further comprises:
a first power terminal, set on the support frame and electrically connected to a power supply of the microfluidic control detection system; and
a second power terminal, set on the bracket and electrically connected to the oscillator; and
the first power terminal and the second power terminal are configured to maintain elastic contact when the bracket is at a position between its lowest position where the bracket enables the sample cup supported on the weighing platform and the position where the bracket lifts the sample cup to the predetermined position, connecting the oscillator to the power supply, and configured to separate when the bracket lifts the sample cup from the predetermined position further upwards, disconnecting the oscillator from the power supply.

6. The microfluidic control detection system according to claim 2 or 3, **characterized in that** the microfluidic control detection system further comprises a lifting mechanism for driving the bracket to move up and move down, which comprising:
a lifting motor, configured to output driving force; and
a drive screw, set vertically and connected to an output shaft of the lifting motor, to rotate under the drive of the lifting motor; wherein
the bracket is mounted on the drive screw and moves up and moves down along the drive screw as it rotates.

7. The microfluidic control detection system according to claim 6, **characterized in that** the lifting mechanism further comprises:
a nut, mounted on the drive screw and connected to threads of the drive screw to move up and move down along the drive screw as it rotates; wherein
the bracket is fixedly connected to the nut, to drive the bracket to move up and move down through the nut.

8. The microfluidic control detection system according to claim 6, **characterized in that** the lifting mechanism further comprises:
a guide rod, set beside the drive screw and extending vertically; wherein
the bracket is mounted on the guide rod, to guide the up movement and down movement of the bracket.

9. The microfluidic control detection system according to claim 6, **characterized in that** the lifting mechanism further comprises:
a limit switch, set near the top of the lead screw, to cause the lifting motor to stop operating when the support frame moves up to touch the limit switch; and
a position of the limit switch is set so that when the lifting motor stops operating triggered by the limit switch, the sample cup on the bracket is at the highest position.

10. The microfluidic control detection system according to any one of claim 1-3, **characterized in that**:
the suction port located on a side surface parallel to a width direction and a length direction of the microfluidic biochip; and the microfluidic control detection system further comprises:
a sample liquid driving device, configured to be in communication with the suction port of the microfluidic biochip via a sealing connector after the microfluidic biochip is in its installed position, to prompt sample liquid in contact with the inlet to enter the microchannels and flow towards the detection pool under the control of the microfluidic biochip; and
a pressing mechanism, configured to apply pressure perpendicular to the side surface to the sealing connector after the microfluidic biochip is installed in its position, to form a fluidic seal connection between the sealing connector and the suction port.

11. A refrigerator, **characterized in that** the refrigerator comprises the microfluidic control detection system according to any one of claims 1-10.
